(19) Europäisches Patentamt
European Patent Office
Office européen des brevets

(11) Publication number: 0 278 501 A2

# (12) EUROPEAN PATENT APPLICATION

(21) Application number: 88101983.0

(22) Date of filing: 11.02.88

(51) Int. Cl.4: C07F 9/65 , C07D 473/30 , C07D 233/86 , C07D 473/18 , A61K 31/52 , A61K 31/415

(30) Priority: 12.02.87 JP 30001/87
25.12.87 JP 331567/87

(43) Date of publication of application: 17.08.88 Bulletin 88/33

(84) Designated Contracting States: AT BE CH DE ES FR GB GR IT LI LU NL SE

(71) Applicant: Takeda Chemical Industries, Ltd.
27, Doshomachi 2-chome Higashi-ku
Osaka-shi Osaka, 541(JP)

(72) Inventor: Fukuda, Tsunehiko
10-202, 9 Oharanonishisakaidani-cho
Nishikyo-ku Kyoto 601-11(JP)
Inventor: Marumoto, Ryuji
53-1, Okuikeminami-machi
Ashiya Hyogo 659(JP)

(74) Representative: von Kreisler, Alek, Dipl.-Chem. et al
Deichmannhaus am Hauptbahnhof
D-5000 Köln 1(DE)

## (54) Nucleotide analogs, their production and use.

(57) The carbocyclic nuucleotides represented by the formula

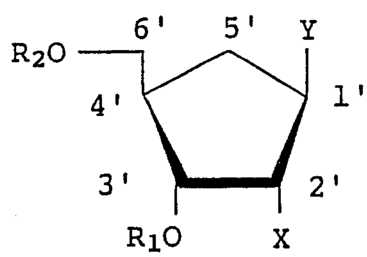

wherein Y stands for a purine base residue or 5-amino-4-carbamoyl-imidazol-1-yl; X stands for hydrogen or an optionally protected hydroxyl group provided that, when Y is adenin-9-yl; X is hydrogen; $R_1$ and $R_2$ independently or combinedly represent phosphoryl group; which shows antiviral action.

EP 0 278 501 A2

## Nucleotide Analogs, Their Production and Use

This invention relates to carbocyclic nucleotides, a method of preparing same and an antivirus agent. More specifically, the present invention relates to cyclopentane analogs of purine nucleotides useful as reagents for research work in the field of biochemistry or medical science and utilizable as medicines such as antiviral agents.

It has been known that cyclopentane analogs of purine nucleosides, as represented by aristeromycin, show interesting activities in various enzyme systems in which common purine nucleoside takes part [Medicinal Research Review, 6, 1 (1986)]. And, DNA containing a carbocyclic analog of 2'-deoxyadenosine has an advantageous characteristic in respect of the genetic recombination technique, as compared with natural type DNA containing 2'-deoxyadenosine[Nucleic Acids Research, Symposium Series, No. 16, 141 p., (1985)]. On the other hand, as the carbocyclic nucleotide analogs, only aristeromycin-3', 6'-cyclic phosphate and aristeromycin-6'-phosphate have been known [Chem. & Pharm. Bull. 27, 990 (1979)].

As stated above, while, among phosphoric acid derivatives of carbocyclic purine nucleoside, those of aristeromycin have been known, no substantial investigation has yet been made on phosphoric acid derivatives of other carbocyclic purine nucleoside.

In view of the above-mentioned circumstances, the present inventors have conducted extensive studies for providing novel and useful carbocyclic purine nucleotides to accomplish the present invention.

More specifically, the present invention relates to

1) a compound represented by the formula (I)

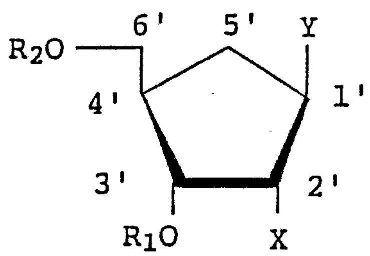

(I)

[wherein Y stands for a purine base residue or 5-amino-4-carbamoyl-imidazol-1-yl(hereinafter called AICA in some instances), X stands for hydrogen or an optionally protected hydroxyl group (provided that, when Y is adenin-9-yl, X is hydrogen), $R_1$, $R_2$ independently stand for hydrogen or a group represented by the formula (A)

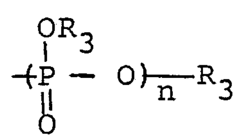

(A)

(wherein n denotes an integer of 1 to 3) (provided that $R_1$ and $R_2$ are not hydrogen simultaneously), or $R_1$ and $R_2$, combinedly, form a group of the formula

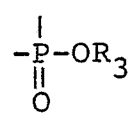

(wherein $R_3$ stands for hydrogen or a hydrocarbon residue having 1 to 14 carbon atoms, and, when two or more $R_3$'s exist, they may be different from one another)], and a salt thereof,

2) a method of preparing a compound of the formula (I), characterized by subjecting a compound represented by the formula (II)

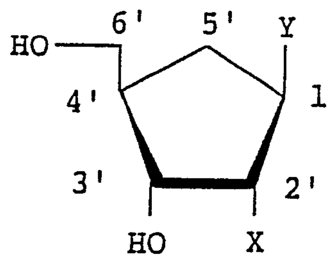

(II)

[wherein Y stands for a purine base residue or 5-amino-4-carbamoyl-imidazol-1-yl, X stands for an optionally protected hydroxyl group (provided that, when Y is adenin-9-yl, X is hydrogen)] to phosphorylation, and

3) An antivirus agent containing a compound represented by the formula (I-1)

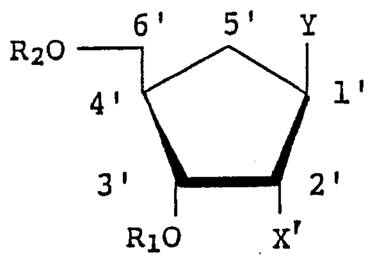

(I-1)

[wherein Y stands for a purine base residue or 5-amino-4-carbamoyl-imidazol-1-yl, X' stands for hydrogen or hydroxyl group (provided that, when Y is adenin-9-yl, X is hydrogen), $R_1$ and $R_2$ independently stand for hydrogen or a group represented by the formula

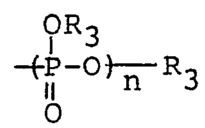

(n denotes an integer of 1 to 3) (provided that $R_1$ and $R_2$ are not hydrogen simultaneously), or $R_1$ and $R_2$, combinedly form a group of the formula

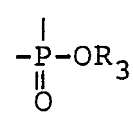

(wherein $R_3$ stands for hydrogen or a hydrocarbon residue having 1 to 14 carbon atoms, and, when two or more $R_3$'s exist, they may be different from one another)], and salts thereof.

In the compounds of the formulas (I), (I-1) and (II), as the purine base residue represented by Y, mention is made of various bases having a skeleton which is called purine ring in the field of nucleoside chemistry. These bases are exemplified by adenine, hypoxanthine, guanine, isoguanine, xanthine, 3-deaza-adenine, 7-deaza-adenine, 8-aza-adenine, 2,6-diaminopurine, etc. Among them, purine bases which are contained in a natural nucleic acid as a constituent are preferably exemplified. These purine bases include adenine, hypoxanthine, guanine, xanthine and 2,6-diaminopurine. Further, such bases as having alkoxy methyl group (represented by $R_4$ mentioned later) at $N^1$-position of the purine ring are also included. In the formula (I), (I-1) and (II), these bases are bonded through nitrogen atom at 9-position of the purine ring.

In the compounds of the formula (I), (I-1) and (II), purine base residues may have protective groups. As the protective groups, i.e. amino-protecting groups at 2-or 6-position, use is made of those commonly employable in the field of nucleoside chemistry. For example, as adenine-protecting groups, use is often made of aromatic carboxylic acid residue of 5 to 30 carbon atoms (e.g. benzoyl), and, as guanine-protecting groups, use is often made of straight or branched aliphatic carboxylic acid residue of 2 to 10 carbon atoms (e.g. acetyl isobutyryl, propionyl).

In the compounds of the formula (I) and (II), as protecting groups when X is a hydroxyl-protecting group, use is made of any ones employable as hydroxyl-protecting groups in the field of nucleoside chemistry with no specific limitation. Examples of these protective groups include, among others, alkyl silyl of 3 to 10 carbon atoms (e.g. t-butyldimethylsilyl), alkyl or alkoxy cyclic ether of 4 to 10 carbon atoms [e.g. tetrahydrofuranyl and tetrahydrofuranyl derivatives of 4 to 7 carbon atoms, tetrahydropyranyl and tetrahydropyranyl derivatives of 5 to 8 carbon atoms (e.g. methoxytetrahydropyranyl)], alkoxy alkyl of 3 to 10 carbon atoms (e.g. ethoxy ethyl, methoxy ethyl), trityl and its alkoxy-substituted compounds (e.g. monomethoxy trityl, dimethoxy trityl), etc. When the protecting group is acyl group, it may be in the form of aliphatic acid ester (e.g. chain-like or branched having 1 to 10 carbon atoms) or of aromatic carboxylic acid ester (e.g. having 5 to 30 carbon atoms). Introduction or removal of these hydroxyl protecting groups may be performed by conventional methods.

Then, examples of hydrocarbon residues of 1 to 14 carbon atoms represented by $R_3$ in the formula (I) and (I-1) include alkyl of 1 to 6 carbon atoms (e.g. methyl, ethyl, propyl, butyl, isobutyl), optionally substituted phenyl groups or aralkyl groups having 7 to 14 carbon atoms (e.g. phenethyl, benzyl). Substituents of phenyl groups are exemplified by methyl, ethyl, chlorine, trifluoromethyl, dimethylamino, amino, methoxy, ethoxy, hydroxyl, etc.

The compound (I) is obtained by subjecting the compound (II) to phosphorylation. On this respect, explanation is given as follows.

1) A compound of the formula (I) wherein $R_1$ has a group represented by the formula (A) and $R_2$ is hydrogen (hereinafter simply referred to as 3′-phosphoric acid ester in some instances) can be prepared by first protecting the primary hydroxyl group at 6′-position using a protective group such as trityl, then subjecting to phosphorylation with a suitable phosphorylating agent, followed by removing the protecting group at 6′-position. The phosphorylation can be carried out by reacting the protected compound with a phosphorylating agent at 10 to 30°C for 30 min. to 10 hours, followed by subjecting hydrolysis in ice water. The phosphorylating agent is normally used at about 2.5 to 10 times the theoretical amount in number of moles.

2) For preparing a compound wherein $R_2$ has a group represented by the formula (A) and $R_1$ is hydrogen (hereinafter simply referred to as 6′-phosphoric acid ester in some instances), the hydroxyl group at 6′-position may be subjected to phosphorylation without protection, though the phosphorylation can be conducted after protecting the hydroxyl group at 3′-position. This is because the hydroxyl group at 6′-position is a primary one and very susceptible to phosphorylation as compared with a secondary hydroxyl group. Acyl type protective groups (e.g. acetyl) are used advantageously for this protection. The phosphorylation can be carried out by reacting with a phosphorylating agent at -40°C to 10°C for 30 min. to 10 hours, followed by subjecting hydrolysis in ice water. The phosphorylating agent is normally used at about 1.2 to 1.5 times the theoretical amount in number of moles.

3) On the other hand, a compound wherein both $R_1$ and $R_2$ are represented by the formula (A) (hereinafter referred to simply as 3′, 6′-diphosphoric acid ester in some instances) can be obtained by subjecting an unprotected starting compound formula (II)] to phosphorylation with an excess amount (e.g. 2.5 to 10 times the theoretical amount in number of moles) of a phosphorylat ing agent or at elevated reaction temperatures (30 to 60°C) for 30 min. to 10 hours and hydrolyzing in ice water.

Examples of phosphorylating agents employable for the phosphorylation usually include those of such a type as directly introducing phosphoric acid residue, e.g. phosphorus oxychloride, pyrophosphoryl tetrachloride, phosphorus trichloride, polyphosphoric acid, metaphosphoric acid, etc., or including steps of removing protecting groups of phosphoric acid after phosphorylation or oxidation process, e.g. phosphoric benzyl ester•dichloride, morpholinophosphoric dichloride, phenyl phosphoric dichloride, di-β-cyanoethyl phosphoric chloride, dibenzyl phosphoric chloride, O-benzyl-phosphite-O,O-diphenyl pyrophosphoric acid, etc. These can be used in any of the reactions set forth in 1) to 3) above. Among phosphorylating agents mentioned above, orthophosphoric acid chlorides or pyrophosphoric acid chlorides are preferably employed in the present phosphorylation.

4) Preparation of a compound (I) wherein $R_1$ and $R_2$ combinedly stand for

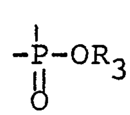

(wherein $R_3$ is of the same meaning as defined above) (hereinafter referred to simply as 3′,6′-cyclic phosphate in some instances):

This compound can be obtained by subjecting the 3'-phosphoric acid ester or the 6'-phosphoric acid ester obtained in the above 1) to 2) to cyclization. For this cyclization, any reaction means can be employed so long as this purpose is attained. This cyclization can be conducted by starting from 6'-phosphoric acid ester or a reactive derivative thereof at its phosphoric acid group after the manner of preparing aristeromycin-3', 6'-cyclic phosphate disclosed in Japanese unexamined patent application No. 50-40590 [processes (1) and (2) mentioned below], a method analogous to the method of synthesizing nucleoside 3', 5'-cyclic phosphate disclosed in Chem. Pharm. Bull., 23, 2295(1975) [process (3) mentioned below] or any other method [Process (4) mentioned below], etc.

(1) 6'-Phosphoric acid ester is subjected to 3',6-cyclization of the phospharic acid portion by direct dehydration. As the dehydrating agent in this case, use is conveniently made of, for example, dicyclohexylcarbodiimide, cyclohexyl isocyanate, inamine derivative [e.g. diethyl(phenylethynyl)amine] or triphenyl phosphine together with 2,2'-dipyridyl disulfide, which are known as utilizable for dehydration of adenoshine-5'-phosphate. As the typical example, detailed explanation is given below on the case of subjecting 6'-phosphoric acid ester to dehydration using dicyclohexyl carbodiimide. Namely, it is convenient to subject a pyridine solution or a mixture solution of pyridine and dimethylformamide, or a pyridine suspension of ammonium salt of 6'-phosphoric acid ester (e.g. tri-n-butyl ammonium salt or morpholino-N,N'-dicyclohexylcarboxy amidinium salt), to boiling together with a pyridine solution of at least 2 mole equivalents of dicyclohexyl carbodiimide. Thus, 3',6'-cyclic phosphoric acid ester is produced.

(2) A reactive derivative of 6'-phosphoric acid ester at the phosphono group is processed with a base to convert into 3',6'-cyclic phosphoric acid ester. This procedure can be conducted after a per se known method of preparing adenosine-3',5'-cyclic phosphate by leading adenosine-5'-phosphate to its reactive derivative, followed by processing the same with a base to give adenosine-3',5'-cyclic phosphate. In accordance with, for example, the method described in "Journal of Organic Chemistry" Vol. 31, pp.3247-3253(1966), a phenol having 1 to 2 nitro groups, e.g. p-nitrophenol, 2,4-dinitrophenol, etc. is allowed to act on the starting compound to give 6'-nitrophenyl phosphoric acid ester, followed by subjecting the compound to de-esterification by processing with a base such as potassium salt of tertiary butanol, thus 3',6'-cyclic phosphoric acid ester can be prepared. Alternatively, 6'-phosphoric acid ester is led to 6'-diphosphoric acid ester or 6'-triphosphoric acid ester compound by a similar method as known in the field of nucleoside chemistry, which comprises reacting the starting ester compound with 1-fluoro-2,4-dinitrobenzene or carbonyl diimidazole at -20°C to 10°C to give the activated 6'-phosphate compound and subjecting to the reaction with an organic amine salt of orthophosphoric acid or pyrophosphoric acid. The resulting ester compound is then processed with a base such as hydroxide of an alkali metal or an alkaline earth metal (e.g. sodium hydroxide, barium hydroxide, etc.) or a weak base thereof (e.g. barium hydroxide, etc.), thus 3',6'-cyclic phosphoric acid ester can be obtained. Alternatively, 6'-phosphoric acid ester is led to $P^1$, $P^2$-pyrophosphoric acid, which is allowed to react with dicyclohexylcarbodiimide in pyridine, thus 3', 6'-cyclic phosphoric acid ester can be produced. Alternatively, 6'-phosphoric acid ester is allowed to react with 1-fluoro-2,4-dinitrobenzene, then resulting 6'-phosphorofluroridate is processed with a base such as potassium salt of tertiary butanol to thereby obtain 3',6'-cyclic phosphoric acid ester.

(3) It is also possible that a compound of the formula (II) is allowed to react with trichloromethanephosphonic acid dichloride in a solvent of relatively high polarity, e.g. pyridine, dimethylformamide, diethyl phosphate, dimethyl sulfoxide, acetone etc., and the resultant 3'-or 6'-trichloromethylphosphonate can be cyclized by processing with a strong base (e.g. tertiary butanol potassium salt).

(4) Further, it is also possible that 3'-or 6'-phosphoric acid ester is led to, for example, imidazolide, which was then condensed with amine salt of pyrophosphoric acid to give triphosphoric acid ester, which is subjected to hydrolytic conditions to afford 3', 6'-cyclic phosphoric acid ester. The process can be carried out in accordance with a known method [The Journal of the American Chemical Society, 79, 3607 (1957)].

On the other hand, in the case of a compound (I), wherein Y is 5-amino-4-carbamoyl-imidazol-1-yl, it is possible that a compound (II) wherein Y is the above-mentioned group is subjected to the same reaction as in the case of having the above-mentioned purine base residue to lead to cyclic phosphoric acid, and also that $N^1$-position of the purine ring of a compound (I) wherein Y is hypoxanthin-9-yl is subjected to alkoxymethylation to cleave the purine ring of a compound (I')

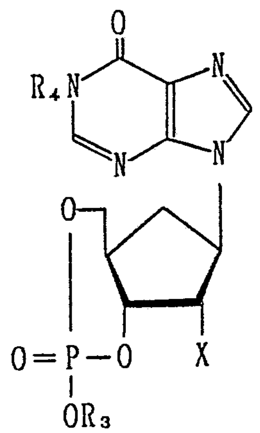

(I')

[wherein X and $R_3$ are of the same meaning as defined above, and $R_4$ stands for alkoxymethyl group] to thereby to lead to cyclic phosphoric acid.

Alkoxymethyl groups represented by $R_4$ in the general formula (I') are generally exemplified by those having 2 to 7 carbon atoms, such as methoxymethyl, ethoxymethyl, phenoxymethyl, etc., and especially methoxymethyl is advantageous. For the above-mentioned alkali treatment, alkali hydroxides are preferably employed, and this treatment can be performed by conducting hydrolysis in, for example, 0.1m to 5M aqueous solutions of sodium hydroxide or potassium hydroxide, or a mixture of such an alkali solution and a neutral aqueous solution of an organic solvent (methanol, ethanol, butanol, dioxane, tetrahydrofuran, etc.) at temperatures ranging from about 10°C to 200°C. The reaction time ranges usually from 10 minutes to 50 hours.

A compound of the formula (I) wherein X is hydrogen (i.e. a compound of the general formula (I-1)] can also be obtained by subjecting a compound of the formula (II) wherein X is a protected hydroxyl group to phosphorylation mentioned as above, then, upon necessity, protecting other hydroxyl groups, removing the hydroxylprotecting group at X-position, then thiocarbonylation or leading to phenylthioformic acid ester, followed by 2'-deoxylation radically using organotin hydride. In this case, the method described on Journal of Organic Chemistry, 47, 485(1982) can be utilized.

The respective phosphoric acid derivatives synthesized by the afore-mentioned reactions can be purified by, after desalting by using charcoal powder, subjecting to chromatography using anion-exchange cellulose or anion-exchange resin. These phosphoric acid derivatives can be obtained, after purification, in the free state, but, when desired, can be isolated as potassium, sodium, calcium, barium and ammonium salts or organic amine salts. A hydrocarbon represented by $R_3$ is then introduced. For this introduction, an alkylating agent is used in general, and, preferably, allowing diazoalkane (e.g. diazomethane, diazobenzyl, etc.) to react in an organic solvent or a mixture of an organic solvent and water.

On the other hand, a substituted phenyl can be introduced by reacting the phosphoric acid residue activated by an activeating agent such as mesytylenesulfonyl chloride with phenols.

Next, a compound of the formula (II) which is employed for production of a compound of the formula (I) of this invention can be obtained by methods described on Chem. Pharm. Bull. 24, 2624(1976), Nucleic Acids Symposium Series, No. 16,141(1985) or Japanese Patent Application No. 61-190830 (Japanese Published Unexamined Patent Application No. 62-174097).

The phosphoric acid esters obtained by the present invention are, as compared with the starting compounds thereof, higher in water-solubility, and their aqueous solutions of relatively higher concentrations can be obtained, thus being of advantage for the use as, for example, anti-viral agents. Further, the compounds (I), because of their being phosphorylated, are shifted to a different metabolic path way and have possibility of presenting a wide variety of phisiological activities. The compounds of this invention are, as compared with aristeromycin 6'-phosphoric acid ester or 3', 6'-cyclic phosphate, low in cytotoxicity, thus facilitating the application as medicines.

The compounds of the formula (I) of the present invention are, as carbocyclic analogs of nucleoside phosphoric acid esters, useful as means of research work in the biochemical field, for example, reagents for research work, and, in the field of medical science, they have possibilities of being used as antiviral agents, anti-fungal agents and anti-protozoal agents, and, besides they are expected to have an anti-tumor effect.

On the other hand, the compounds of formula [I-1] according to the present invention have antiviral activity against herpes virus type I or II, adenovirus or vacciniavirus, for instance. The compounds can be used as antiviral agent for the treatment of virus-induced disease in animals, particularly in mamalian

animals (laboratory animals such as rabbit, rat and mouse; pet animals such as dog and cat; and human being).

For use in the treatment mentioned above, the compounds of the present invention can be orally or otherwise administered, either as it is or in combination with an appropriate pharmacologically acceptable carrier, excipient or diluent, in such dosage forms as powders, granules, tablets, capsules, solutions, ointments and injections. There may be mentioned as carrier lactose, starch, mineral oil, petroleum jelly, polyethyleneglycol, propyleneglycol, saline solution for injection on the like. The dosage depends on the kind of virus, symptoms, administration hosts and routs or the like. For the treatment of herpes virus-induced disease in an adult human being, for instance, the compound is desirably administered in a vein at a daily dose level of about 1 to 50 mg in a single dose or up to 3 divided portions. For the oral route, the compound is desirably administered at a level of about 10 to 100 mg per a dose in a single or up to 3 divided portions.

A compound of the formula (I-1) may be administered intravenously in a higher concentration in term of its high water-solubility according to the introduction of phosphate residue. Further, compounds (I-1) have a possibility to show effectiveness against a resistant strain of herpes simplex virus. Among them, as antiviral agents, compounds (I-1) wherein X is hydrogen are preferable, and those wherein X is hydrogen and, at the same time, which are 6′-monophosphate and 6′-triphosphate are utilizable more advantageously.

The following working examples, reference examples and test examples illustrate the present invention more specifically.

Example 1

2′-Deoxyaristeromycin-3′,6′-cyclic phosphate

2′-Deoxyaristeromycin (249 mg, 1 mmol.) was dissolved in triethyl phosphate (10 ml). To the solution was added dropwise phosphorus oxychloride (0.3 ml) under ice-cooling. The mixture was stirred at the same temperature for two hours. The reaction solution was poured to ice-water (100 ml), followed by neutralization with triethylamine. Thus-neutralized solution was allowed to pass through a column of activated chacoal powder (3 g). The column was washed with water and eluted with an eluent (water/ethanol/triethylamine = 10/9/1, 500 ml). The eluate was concentrated to dryness. The concentrate was dissolved in pyridine, which was subjected to dehydration by means of azeotropic distillation. Thus-obtained 2′-deoxyaristeromycin - monophosphate•triethylammonium salt was dissolved in pyridine (100 ml). The solution was added dropwise taking 2 hours to a solution of DCC (820 mg, 4 mmol.) in boiling pyridine (100 ml). The mixture was boiled for further one hour. The reaction solution was concentrated to dryness, and the concentrate was dissolved in a mixture of water/ether(1/1, 200 ml), and the aqueous layer then separated was subjected to extraction twice with ether (100 ml). The aqueous layer was concentrated under reduced pressure to a volume of 80 ml. The concentrate was applied to a column of DEAE-cellulose($HCO_3$-form, 80 ml). The column was washed with water (500 ml), followed by elution with 0.05M ammonium hydrogencarbonate (500 ml). The eluate was concentrated to dryness, and the concentrate was dissolved in a small volume of methanol. To the solution was added acetone, then precipitating white powder (180 mg) was collected by filtration and dried.

Elemental Analysis (%) : $C_{17}H_{19}N_6O_4P \bullet H_2O$ : (molecular weight 420.35)

Calcd. : C; 48.57, H; 5.03, N; 19.99, P; 7.36

Found : C; 48.32, H; 5.48, N; 19.41, P; 7.04

UV absorption spectrum ; $\lambda_{max}^{pH\ 7}$ :259 nm

The present compound is changed to 2′-deoxyaristeromycin-mono phosphate by reacting with cyclic AMP phosphodiesterase originated from a cow brain.

Example 2

Carbocyclic•guanosine-3′,6′-cyclic phosphate

9-[(1R,2S,3R,4R)-4-hydroxymethyl-2,3-dihydroxylcyclopentan-1-yl]-guanine(263 mg, 1 mmol.) was subjected to a reaction similar to that in Example 1 to give triethylammonium salt of carbocyclic•guanosine-3′,6′-cyclic phosphate, which was dissolved in water (50 ml). The solution was allowed to pass through a column of IR-120 ($Na^+$ form, 10 ml) and concentrated to dryness under reduced pressure. The concentrate

was dissolved in methanol, to which was added acetone, then precipitating powder (280 mg) was collected by filtration.

Elemental Analysis (%) : $C_{11}H_{13}N_5O_6PNa \bullet 1/2H_2O$ (molecular weight 374.25)

Calcd. : C; 35.30, H; 3.76, N; 18.71, P; 8.28

Found : C; 34.87, H; 4.21, N; 18.47, P; 8.03

UV absorption spectrum ; $\lambda_{max}^{pH\ 7}$ :252 nm

Example 3

Carbocyclic•2'-deoxyguanosine-3',6'-cyclic phosphate

9-[(1R,3S,4R)-4-hydroxymethyl-3-hydroxylcyclopentan-1-yl] guanine (247 mg, 1 mmol.) was subjected to a reaction similar to that in Example 1 to give once the corresponding ammonium salt, which was dissolved in water (30 ml). The solution was adjusted to pH 2 with N hydrochloric acid, whereupon colorless crystals (150 mg) precipitated out.

Elemental Analysis (%) : $C_{11}H_{14}N_5O_5P$ molecular weight 327.26)

Calcd. : C; 40.37, H; 4.31, N; 21.39, P; 9.47

Found : C; 40.01, H; 4.53, N; 20.84, P; 9.03

UV absorption spectrum ; $\lambda_{max}^{pH\ 7}$ :252 nm

The properties of the corresponding triethyl ammonium salt are as follows:

m.p. 285 - 287°C

Elemental Analysis (%) : $C_{17}H_{29}N_6O_5P$ molecular weight 428.46)

Calcd. : C; 47.65, H; 6.82, N; 19.61

Found : C; 47.33, H; 6.96, N; 19.47

Example 4

Carbocyclic•AICA•riboside-3',6'-cyclic phosphate

Aristeromycin-3', 6'-cyclic phosphate (327 mg, 1 mmol.) was dissolved in 5% acetic acid (40 ml). To the solution was added sodium nitrite (2 g), and the mixture was left standing at 20°C for 20 hours. To the reaction solution was added water (50 ml), and the mixture was allowed to pass through a column of activated charcoal (10 g). The column was washed with water (500 ml), followed by elution with the same solvent (600 ml) as in the case of Example 1. The eluate was concentrated to dryness. The concentrate was suspended in pyridine (10 ml), to which was added acetic anhydride (1.5 ml). The mixture was stirred at room temperature for 20 hours to give a complete solution. The reaction solution was concentrated to dryness, which was dissolved in 50% methanol. The solution was allowed to pass through a column of IR-120 ($NH_4^+$ form, 10 ml). The passing solution was concentrated to dryness, and the concentrate was dissolved in a dioxane/dimethylformamide(3:1) mixture solution (10 ml). To the solution was added sodium hydride (60 mg) under nitrogen streams. To the mixture was added under ice-cooling methoxymethylchloride (90 $\mu$l), and the mixture was stirred at room temperature for two hours. The reaction solution was concentrated to dryness, and the concentrate was dissolved in dichloromethane. The solution was washed with 0.1M triethylammonium hydrogencarbonate, and the dichloromethane layer was concentrated to dryness. The concentrate was dissolved in ethanol (15 ml). To the solution was added 1M sodium hydroxide (5 ml), and the mixture was stirred at 25°C for 40 hours. The reaction solution was neutralized, to which was added water (100 ml). The mixture was allowed to be adsorbed on a column of DEAE cellulose ($HCO_3^-$ form, 80 ml), and the column was washed with water (500 ml). Main fractions eluted with 0.05M ammonium hydrogencarbonate were combined and concentrated to dryness. The concentrate was dissolved in a small volume of methanol. To the solution was added acetone, then precipitating white powder (105 mg) was collected by filtration.

Elemental Analysis (%) : $C_{10}H_{18}N_5O_6P \bullet 2H_2O$ (molecular weight 371.28)

Calcd. : C; 32.35, H; 5.96, N; 18.86, P; 8.35

Found : C; 31.87, H; 5.99, N; 18.42, P; 8.07

UV absorption spectrum ; $\lambda_{max}^{pH\ 7}$ : 268 nm

Example 5

Carbocyclic•guanosine-6'-triphosphate

9-[(1R,2S,3R,4R)-4-hydroxymethyl-2,3-dihydroxylcyclopentan-1-yl]guanine (26 mg, 0.1 mmol.) was subjected to a reaction similar to that in Example 1, followed by purification by means of a column of DEAE-cellulose to afford 6'-phosphoric acid ester of 0.07 mmol. This product was suspended in hexamethylphosphoramide (5 ml). To the suspension was added carbonyldiimidazole (50 mg), and the mixture was stirred to dissolve completely. Four hours later, methanol (20 µl) was added to the solution, and the reaction was suspended. To the resultant was added a hexamethylphosphoramide solution (5 ml) of tetraquis (tributylammonium) pyrophosphate (300 mg), and the mixture was stirred at room temperature) for 40 hours. The reaction solution was poured to water (100 ml), which was allowed to be adsorbed on DEAE cellulose (1.5 cm × 10 cm), followed by washing with water. The fraction (320 µmol.) eluted with 0.3M triethylammonium hydrogencarbonate(TEAB) was concentrated to dryness, followed by lyophilization again to obtain a powdery product.

Elemental Analysis (%) : $C_{35}H_{78}N_9O_{13}P_3 \bullet 2H_2O$ (molecular weight 961.97)

Calcd. : P; 9.65

Found : P; 10.24

UV absorption spectrum ; $\lambda_{max}^{pH\ 1}$ :256 nm, $\lambda_{max}^{pH\ 7}$ : 252 nm, $\lambda_{max}^{pH\ 12}$ :258 nm

Example 6

Carbocyclic•guanosine-3',6'-cyclic phosphate

Carbocyclic•guanosine-6'-triphosphate obtained in Example 5 was subjected to the treatment at temperatures ranging from 0°C to 120°C in an aqueous solution of barium hydroxide or a methanol•pyridine mixture solution of sodium hydroxide to afford the substance obtained by the method described in Example 2, in a yield of 30%.

Example 7

Carbocyclic•2'-deoxyguanosine-6'-monophosphate

9-[(1R,3S,4R)-4-hydroxymethyl-3-hydroxylcyclopentan-1-yl] guanine (40 mg) was subjected to phosphorylation in a manner similar to that in Example 1 with triethyl phosphate (1 ml) and phosphoryl oxychloride (15 µl), followed by purification (elution with 0.1M TEAB) with a column of DEAE cellulose (Ø1.5 cm × 10 cm) to afford 0.04 mmol. of carbocyclic•2'-deoxyguanosine-6'-monophosphate. m.p. 283 - 285°C

Elemental Analysis (%) : $C_{11}H_{16}N_5O_7P$ (molecular weight 363.25)

Calcd. : C; 36.37, H; 4.99, N; 19.27, P; 8.52

Found : C; 35.94, H; 5.32, N; 19.63, P; 8.10

Example 8

Carbocyclic•2'-deoxyguanosine 6'-triphosphate

Carbocyclic•2'-deoxyguanosine-6'-monophosphate (80 mg) was dissolved in hexamethylphosphoramide (1 ml), to which was added carbonyldiimidazole (36 mg, 0.22 mmol.). The reaction was allowed to proceed for 4 hours, followed by addition of methanol (25 µl) to suspend the reaction, followed by allowing the reaction to proceed in a manner similar to that in Example 5 to afford 6'-triphosphate. Purification of this product with DEAE cellulose gave the desired product of 0.025 mmol. by using about 150 ml of 0.2M TEAB.

Elemental Analysis (%): $C_{35}H_{78}N_9O_{12}P_3 \bullet 3H_2O$ (molecular weight 964.02)

Calcd.: P; 9.64

Found : P; 9.21

Example 9

Carbocyclic•2'-deoxyguanosine-3',6'-cyclic phosphate

Carbocyclic•2'-deoxyguanosine-6'-triphosphate obtained in Example 8 was processed in a manner similar to that in Example 6 gave the same product as obtained in Example 3.

Example 10

(1) Tablets for oral administration of the antiviral composition according to the present invention are prepared as exemplified below:

Carbocyclic•2'-deoxyguanosine-6'-monophosphate (the compound of Example 7) (20 mg), 250 mg of lactose, 50 mg of starch and 2 mg of magnesium stearate are mixed in methanol and after removal of methanol with heating is molded into tablets.

(2) Ointment for the antiviral composition are prepared as exemplified below:

Carbocyclie•2'-deoxyguanosine-6'-monophosphate (the compound of Example 7) (0.1 g), 40.0 g of vaselinum album, 18.0 g of ethanol, 5.0 g of sorbitan-sesquioleate, 0.5 g of polyoxyethylenelauryl-alcohol-ether, 0.1 g of methyl-p-hydroxybenzoate and 36.3 g of purified water are mixed to prepare 0.1% ointments.

(3) Injection agent:

Carbocyclic•2'-deoxyguanosine-6'-monophosphate (0.1 g) is dissolved in pyrogen-free sterile phosphate buffer (pH 8.0, 10 ml), and the solution is sealed up in a vial through a micropore filter.

Reference Example 1

Synthesis of 9-[(1R,2S,3R,4R)-4-methyl-2-hydroxy-3,6-(tetraisopropyldisiloxanyl)dioxy-cyclopentan-1-yl]-hypoxanthine

C-analogue of inosine(10 g, 37.5 mmol.) was dissolved in 200 mℓ of anhdyrous DMF. To the solution were added 1,3-dichloro-1,1,3,3-tetraisopropyldisiloxane(13 mℓ, 41 mmol.) and imidazole(11.3 g, 165 mmol.), and the mixture was stirred at room temperature for 2.5 hours. The reaction solution was added dropwise to 2ℓ of water, then resulting precipitates were collected by filtration, washed with water, and further washed with diethyl ether quickly, followed by drying to obtain a white powdery compound(17.2 g). A portion of the compound was recrystallized from dichloromethane to afford a crystalline product, m.p.135-138°C.

Reference Example 2

Synthesis of 9-[(1R,2S,3R,4R)-4-methyl-2-benzoylthiocarbonyloxy-3,6-(tetraisopropyldisiloxanyl)-dioxycyclopentan-1-yl]hypoxanthine

The compound obtained in Reference Example 1(11.2 g, 22.3 mmol.) was dissolved in 300 mℓ of anhdyrous acetonitrile. To the solution were added dimethylaminopyridine(15.8 g, 53.5 mmol.) and phenoxythiocarbonyl chloride(5 g, 29 mmol.). The mixture was stirred at room temperature for 7 hours. The solvent was removed from the reaction solution under reduced pressure. The residue was dissolved in 250 mℓ of chloroform. The solution was washed with 0.5 M solution of potassium dihydrogenphosphate(250mℓ × 2)-,then with water (200 mℓ), dried(anhydrous sodium sulfate), followed by concentration under reduced pressure to afford a yellow syrupy substance. Purification of this substance by means of silica gel

chromatography(90 g, solvent;$CHCl_3$ and $CHCl_3$/MeOH = 60/1) afforded a pale yellow glassy compound(13.0 g).

NMR(60MHz,$CDCl_3$)δppm: 1.0-1.23(28H,m), 2.13-2.43(3H,m,H4',H5'),3.93-4.10(2H,m,$H_6$'), 4.80-5.20-(2H,m,$H_1$',$H_3$'), 6.00-6.20(1H,m,$H_2$'), 7.03-7.50(5H,m), 7.87(1H,s), 8.13(1H,s),

Reference Example 3

Synthesis of 9-[(1R,3S,4R)-4-methyl-3,6-(tetraisopropyldisiloxanyl)dioxy-cyclopentan-1-yl]hypoxanthine

To the compound obtained in Reference Example 2(13.0 g, 20 mmol.) was added 30 ml of anhydrous toluene, followed by concentration under reduced pressure. The concentrate was dissolved in 300 ml of anhydrous toluene. Nitrogen gas was bubbled into the solution for 20 minutes. To the resultant was added tributyltin hydride(11 ml, 40 mmol.). To the mixture was added, while heating at 80°C, crystals(820 mg) of α,α'-azobisisobutyronitrile(AIBN) in four portions at a 15 minutes' interval. The mixture was stirred for 3 hours and, then, the solvent was removed under reduced pressure to leave an oily substance, which was purified by means of a silica gel chromatography(80 g, solvent;$CHCl_3$ and $CHCl_3$/MeOH = 60/1 to 30/1) to give a colorless glassy compound(10.4 g). A portion of the compound was recrystallized from ethanol to give colorless needles, m.p.200-202°C.

NMR(60MHz,$CDCl_3$)δppm: 0.93-1.20(28H,s), 1.97-2.53(5H,m,$H_2$', $H_4$', $H_5$'), 3.80-4.07(2H,m,$H_6$'), 4.43-5.27-(2H,m,$H_1$',$H_3$'), 7.87(1H,s), 8.20(1H,s)

Reference Example 4

Synthesis of 9-[(1R,3S,4R)-4-(monomethoxythyroxy)methyl-3-hydroxyl-cyclopentan-1-yl]-(1-methoxy-methyl-hypoxanthine)

The compound obtained in Reference Example 3(98 g, 19.8 mmol.) was dissolved in 240 ml of anhydrous dioxane. To the solution was quickly added, while stirring under ice-cooling, sodium hydride(880 mg, 21.8 mmol.). The mixture was, after its temperature was reverted to room temperature, stirred for 1.5 hour, followed by adding quickly, under ice-cooling, methoxymethyl chloride(2 ml, 21.8 mmol.). The mixture was further stirred at room temperature for 3 hours.

From the reaction mixture was removed the solvent under reduced pressure to leave an oily substance, which was dissolved in 200 ml of chloroform. The chloroform solution was washed with a 0.1M triethyl bicarbnate(TEAB) buffer solution (pH 7.5, 100 ml × 2) then with water(200 ml), followed by drying-(anhydrous sodium sulfate). The resultant was concentrated under reduced pressure to afford a syrupy substance. The syrupy substance was purified by means of a $C_{18}$ silica gel chromatography(ø5.3 × 7.0 cm, solvent; acetone-water, 55%-80%) to afford a colorless glassy compound(8.5 g).

This compound(8.0 g) was dissolved in 32 ml of tetrahydrofuran(THF). To the solution was added trihydrate of tetra butylammonium fluoride(TBAF•$3H_2O$)(10g), and the mixture was stirred at room temperature for 0.5 hour. From the reaction mixture was removed the solvent under reduced pressure to leave an oily substance. The oily substance was dissolved in 100 ml of water, and the aqueous solution was washed with diethyl ether(100 ml × 2), followed by eliminating tetrabutylammonium salt by allowing it to be adsorbed on Dowex-50(pyridine form, 60 ml) resin. The passing solution and the aqueous washing(240 ml) of the resin were combined and concentrated. The concentrate was subjected to azeotropic distillation with pyridine three times to perform dehydration. The resultant was dissolved in 100 ml of pyridine. To the solution was added monomethoxytrityl chloride(MMTrCl) (5.4 g), and the mixture was stirred at 37°C for 4 hours. The solvent was removed under reduced pressure to leave an oily substance, which was distiributed to a 0.1M-TEAB buffer solution(50 ml) and $CHCl_3$(100 ml). The organic layer was further washed with water-(100 ml), dried(anhydrous sodium sulfate) and concentrated under reduced pressure. The concentrate was subjected to azeotropic distillation with toluene to afford a colorless syrupy substance. On the other hand, the 0.1M-TEAB buffer solution and the aqueous washing were combined and concentrated to recover the non-monomethoxytritylated compound. This compound was concentrated and purified by using HP-20 resin(190 ml, solvent; water and 30% aqueous ethanol), followed by concentration. The concentrate was subjected to azeotropic distillation with pyridine, and monomethoxytritylation was carried out in a manner similar to the above. The purification of the object compound of this Reference Example was performed, after combining the both substances obtained as above, by means of a silica gel chromatography(80 g,

solvent; $CHCl_3$/MeOH = 100/1, 60/1, 50/1) to afford a colorless glassy compound (6.1 g). Further, a portion of the compound was dissolved in dichloromethane, and the solution was added dropwise to n-hexane to thereby afford a white powdery product.

NMR(60MHz,$CDCl_3$)δppm: 1.87-2.70(5H,m,$H_2'$,$H_4'$,$H_5'$), 3.20-3.40(2H,m,$H_6'$), 3.43(3H,s,$C\underline{H}_3OCH_2$), 3.80-(3H,s), 4.30-4.57(1H,m,$H_3'$), 4.87-5.10(1H,m,$H_1'$), 5.47(2H,s,$CH_3OC\underline{H}_2$-N), 6.73-6.97(2H,m), 7.17-7.53(12H,m), 7.73(1H,s), 7.98(1H,s)

Reference Example 5

Synthesis of 1-[(1R,3S,4R)-4-(monomethoxytrityloxy)methyl-3-hydroxyl-cyclopentan-1-yl]-(4-carbamoyl-5-aminoimidazole)

The compound obtained in Reference Example 4 (6.1 g, 10.7 mmol.) was dissolved in 490 ml of ethanol. To the solution was quickly added, while heating under reflux, 130 ml of a 5M aqueous solution of sodium hydroxide previously warmed, and the reflux was continued for further 40 minutes. The solvent was removed under reduced pressure to leave an oily substance, which was dissolved in 200 ml of chloroform, washed with water(100 ml × 2), then with a 0.1M-TEAB buffer solution(100 ml × 2) and further with a saturated aqueous saline solution(100 ml), followed by drying(anhydrous sodium sulfate) and concentration under reduced pressure to afford a syrupy substance. This substance was purified by means of a silica gel chromatography(90 g, solvent; $CHCl_3$/MeOH = 100/1 -20/1) to give a colorless glassy compound(3.2 g). A portion of this compound was dissolved in chloroform, and the solution was added dropwise to n-pentane under stirring to give a white powdery compound.

Elemental Analysis (%) for $C_{30}H_{32}N_4O_4 \bullet 0.5H_2O$ molecualr weight 521.616

Calcd. : C; 69.08, H; 6.38, N; 10.74

Found : C; 69.14, H; 6.09, N; 10.54

NMR(100MHz,$CDCl_3$)δppm: 1.36-2.52(5H,m), 3.00-3.40(3H,m,$H_6'$,OH), 3.77(3H,s), 4.12-4.60(2H,m,$H_1'$,$H_3'$), 4.80-5.28(2H,br.$NH_2$), 5.64-6.44(2H,br,$NH_2$), 6.76-6.94(3H,m), 7.14-7.48(12H,m)

Reference Example 6

Synthesis of 1-[(1R,3S,4R)-4-hydroxymethyl-3-hydroxyl-cyclopentan-1-yl]-(4-carbamoyl-5-aminoimidazole)

The compound obtained in Reference Example 5 (2.3 g, 4.4 mmol.) was dissolved in 50 ml of 80% acetic acid, and the solution was stirred at 40°C for 7 hours. The solution was concnetrated under reduced pressure, and the concentrate was subjected to azeotropic distillation with toluene then with ethanol. The residue was dissoved in 12 ml of ethanol, and the solution was added dropwise under stirring 130 ml of n-hexane-ether(1:1, v/v) to afford a syrupy substance. This substance was purified by means of $C_{18}$silica gel chromatography(10 g, solvent; water and 5% acetone-water). The solvent was removed under reduced pressure, and recrystallization from ethanol was performed to give the object compound (0.93 g) of this Reference Example, m.p.162-163°C.

$\lambda_{max}$nm:($H_2O$)234(sh), 268;($H^+$)244, 269;($OH^-$)267.5

NMR(60MHz,DMSO-$d_6$ + $D_2O$)δppm: 1.67-2.67(5H,m), 3.43-3.60(2H,m,$H_6'$), 3.90-5.00(2H,m), 7.23-(1H,s,$H_2$);(DMSO-$d_6$) 5.77(2H,bs,$NH_2$) , 6.63(2H,bs,$NH_2$)

Elemental Analysis (%) for $C_{10}H_{16}N_4O_3$, molecular weight 240.262

Calcd. : C; 49.99, H; 6.71, N; 23.32

Found : C; 49.32, H; 6.34, N; 22.92

Reference Example 7

Synthesis of 1-[(1R,3S,4R)-4-(monomethoxytrityloxy)methyl-3-hydroxyl-cyclopentan-1-yl]-[4-carbamoyl-5-(N-benzoyl-S-methylisothio-carbamoyl)aminoimidazole]

The compound obtained in Reference Example 5 (0.88 g, 1.7 mmol.) was dissolved in 25 ml of anhydrous acetone. To the solution was added, while heating under reflux, an acetone solution(8 ml) of benzoyl isothiocyanate(260μℓ,1.9mmol.) over 10 minutes, followed by refluxing for 50 minutes. The solvent

was removed under reduced pressure to leave a pale yellow glassy substance, which was purified by means of a silica gel chromatography(15 g, solvent; $CHCl_3$/MeOH = 50/1 - 30/1) to afford a pale yellow glassy compound(0.87 g). To this compound(0.84 g, 1.2 mmol.) was added a small volume of acetone to give a syrupy substance , to which was added 12.5 ml of 0.2N-NaOH. The mixture was subjected to ultrasonic treatment to give a homogeneous solution. To the solution was added, under stirring, dimethylsulfuric acid (130$\mu l$, 1.4 mmol.). The mixture was stirred vigorously at room temperature for one hour. The reaction solution was distributed to $CHCl_3$(15 ml × 2), and the organic layer was washed with a 0.1M-TEAB buffer solution(15 ml × 3), then with a saturated aqueous saline solution(20 ml), and dried (anhydrous sodium sulfate), followed by concentration under reduced pressure. The concentrate was purified by means of a silica gel chromatography(15 g, solvent;$CHCl_3$/MeOH = 100/1 -60/1). To the glassy substance thus-obtained was obtained a small volume of dichloromethane. The mixture was added dropwise to hexane, and precipitates deposited thereby were collected by centrifuge, followed by drying to obtain 400 mg of the object compound of this Example as a powdery product.

Elemental Analysis (%) $C_{39}H_{39}O_5O_5S_1$ molecular weight 689.835

Calcd. : C; 67.90, H; 5.70, N; 10.15

Found : C; 67.45, H; 5.45, N; 9.89

NMR(100MHz,$CDCl_3$),δppm: 1.34-2.60(5H,m), 2.52(3H,s,$SCH_3$), 3.04-3.44(2H,m,$H_6'$), 3.79(3H,s,$OCH_3$), 4.08-4.44(1H,m,$H_3'$), 4.60-5.00(1H,m,$H_1'$), 5.64(1H,bs,$NH_2$), 6.72-6.94(3H,m), 7.12-7.52(15H,m), 7.80-7.96-(2H,m), 11.35(1H,bs,NH)

Reference Example 8

Synthesis of 1-[(1R,3S,4R)-4-hydroxymethyl-3-hydroxyl-cyclopentan-1-yl]-[4-carbamoyl-5-(N-benzoyl-S-methylisothio-carbamoyl)aminoimidazole]

The compound obtained in Reference Example 6 (815 mg, 3.4 mmol.) was allowed to react with 1.1 equivalent of benzoyl isothiocyanate in acetone. The solvent was removed under reduced pressure. To the residue was added 15 ml of acetone-$CHCl_3$(2:1,v/v). To the mixture was added ether, then resulting precipitates were collected by filtration and dried to afford 1.4 g of a powdery product. The product was dissolved in 35 ml of 0.2N sodium hydroxide. To the solution was added dimethyl sulfate(340$\mu l$), and the mixture was stirred at room temperature for one hour. To the reaction solution was added under ice-cooling acetic acid (pH 4-5). The resultant white-turbid reaction solution was subjected to extraction with n-butanol-(20 ml × 3) to obtain the reaction product, which was washed with water(10 ml × 2), followed by removing the solvent under reduced pressure. The product was purified by means of a silica gel chromatography ($C_{18}$ silica gel 10 g, solvent; water and 30% acetone-water) to afford a pale yellow glassy substance(920 mg), which was recrystallized from water to afford the object compound of this Reference Example as colorless crystals(570 mg), m.p.119-120°C.

Elemental Analysis (%) $C_{19}H_{23}N_5S_1O_4$•$0.3H_2O$, molecular weight 422.886

Calcd. : C; 53.96, H; 5.62, N; 16.56, S; 7.58

Found : C; 54.03, H; 5.49, N; 16.44, S; 7.53

NMR(100MHz, DMSO-$d_6$)δppm: 2.52(3H,s,$CH_3$), 7.34-7.94(6H,m), 11.85(1H,bs,NH) were confirmed.

Reference Example 9

Synthesis of 9-[1R,3S,4R]-4-monomethoxytrityloxymethyl-3-hydroxyl-cyclopentan-1-yl]guanine

The compound obtained in Reference Example 7(360 mg, 0.53 mmol.) was added to 18 ml of warmed 6N sodium hydroxide, and the mixture was heated for one hour under reflux. The reaction product was extracted with $CHCl_3$, and the extract was washed with 0.1M-TEAB buffer solution(30 ml), then with a saturated aqueous saline solution(30 ml), and dried (anhydrous sodium sulfate), followed by purification by means of a silica gel chromatography(8g, solvent;$CHCl_3$/MeOH = 40/1-6/1). To the glassy substance thus-obtained was added a small volume of acetone. The mixture was added dropwise to pentane, and resulting precipitates were collected by centrifugal separation, followed by drying to afford 210 mg of the object compound as a powdery prodcut.

Elemental Analysis (%) $C_{31}H_{31}N_5O_4$•$1.0H_2O$, molecular weight 555.633

Calcd. : C; 67.01, H; 5.99, N; 12.60
Found : C; 67.01, H; 5.69, N; 12.42
NMR(100MHz,DMSO-$d_6$)δppm: 1.50-2.60(5H,m), 3.01(2H,bs), 3.98-4.20(1H,m), 4.70-4.96(2H,m), 6.37-(2H,bs,$NH_2$), 6.82-7.46(14H,m), 7.68(1H,s,$H_8$), 10.60(1H,bs,NH)

Reference Example 10

Synthesis of 9-[(1R,3S,4R)-4-hydroxymethyl-3-hydroxyl-cyclopentan-1-yl]guanine

The compound obtained in Reference Example 9 (180 mg, 0.33 mmol) was dissolved in 10 ml of 80% acetic acid, and the solution was stirred at 40°C for 4.5 hours. The solvent was removed under reduced pressure, and the residue was subjected to azeotropic distillation with water twice, followed by addition of 10 ml of water. The mixture was washed with ether(10 ml × 2). Then, water was eliminated under reduced pressure to afford 41 mg of the object compound as colorless crystals, m.p.246-248°C.
$[\alpha]_D^{25}$ = +7.7° (c=0.5, DMF) $\lambda_{max}$ (nm):($H_2O$); 255, 278(sh) ($H^+$) ; 257, 282 ($OH^-$); 256(sh),273
Elemental Analysis (%) $C_{11}H_{15}N_5O_3$•$0.5H_2O$•$0.1C_2H_5OH$ molecualr weight 278.886
Calcd. : C; 48.24, H; 6.00, N; 25.11
Found : C; 48.61, H; 6.41, N; 25.40

Reference Example 11

Synthesis of 9-[(1R,3S,4R)-4-monomethoxytrityloxymethyl-3-hydroxyl-cyclopentan-1-yl]isoguanine

The compound obtained in Reference Example 7 (585 mg, 0.85 mmol.) was dissolved in 10 ml of ethanol. To the solution were added 6 ml of 1N-NaOH and 44 ml of water, and the mixture was heated for 2 hours under reflux. To this reaction solution was further added 60 ml of 0.1N-NaOH, and the mixture was heated for further 2 hours under reflux. This reaction solution was neutralized with 1N-HCl. The reaction product was extracted with $CHCl_3$, followed by washing with a saturated aqueous saline solution then with water. Chloroform was removed under reduced pressure to leave a syrupy substance. This substance was left standing overnight to give the object compound as white crystals(200 mg, m.p.245-247°C). Further, the filtrate was purified by means of a silica gel chromatography(10 g, solvent;50-90% acetone-water).
Element Analysis (%) $C_{31}H_{31}N_5O_4$ molecular weight 537.618
Calcd. : C; 69.26, H; 5.81, N; 13.03
Found : C; 69.80, H; 5.86, N; 12.60
NMR(100MHz,DMSO-$d_6$)δppm: 1.46-2.46(5H,m,2H$_{2'}$,2H$_{5'}$,H$_{4'}$), 3.00-3.38(4H,m), 3.75(3H,s,$CH_3$), 3.96-4.16-(1H,m,H$_{3'}$), 4.68-4.94(2H,m), 6.38(1H,bs,NH),6.80-7.50(14H,m), 7.77(1H,s,$H_8$)

Reference Example 12

Synthesis of 9-[(1R,3S,4R)-4-hydroxymethyl-3-hydroxyl-cyclopentan-1-yl]isoguanine

The compound obtained in Reference Example 12 (148 mg, 0.27 mmol.) was dissolved in 11 ml of 80% acetic acid, and the solution was stirred at 45°C for 6 hours. The solvent was removed under reduced pressure, and the residue was subjected to azeotropic distillation with water. The residual aqueous solution was washed with ether(10 ml × 2). Water was removed under reduced pressure to leave a glassy substance, which was suspended in ethanol to afford the object compound(69 mg, m.p.162-165°C-(decomp.)).
Elemental Analysis (%) $C_{11}H_{15}N_5O_3$•$0.5H_2O$•0.1EtOH molecular weight 278.887
Calcd. : C; 48.24, H; 6.00, N; 25.11
Found : C; 47.92, H; 6.05, N, 24.89
$\lambda_{max}$ (nm): ($H_2O$); 249, 253(sh), 294($H^+$); 236, 242(sh), 283($OH^-$); 250, 285
NMR(100MHz, DMSO-$d_6$+$D_2O$)δppm: 1.10-2.60(5H,m,2H$_{2'}$,2H$_{5'}$,H$_{4'}$), 3.40-3.60(2H,m,2H$_{6'}$,), 4.00-4.20-(1H,m,H$_{3'}$), 4.60-5.10(1H,m,H$_{1'}$), 7.90(1H,s,$H_8$)

Reference Example 13

Synthesis of the compound of Reference Example 10 (an alternate method)

The compound of Reference Example 2 (420 mg) was dissolved in 10 mℓ of 6N sodium hydroxide. The solution was quickly heated and refluxed for one hour. The temperature of the reaction solution was made to room temperature, then the solution was neutralized with 1N hydrochloric acid. This reaction solution was purified by means of an HP-20 chromatography(190 mℓ, solvent; water and 10% aqueous ethanol), followed by concentration to afford colorless crystals(174 mg), m.p.246-248°C.

Reference Example 14

Synthesis of 9-[(1R,2S,3R,4R)-4-monomethoxytriyloxymethyl-2,3-(dimethylmethylene)dioxy-cyclopentan-1-yl] hypoxanthine

C-analog of inosine(10 g, 37.6 mmol.) was suspended in 380 ml of acetone. To the suspension were added 2,2-dimethoxypropane(23 ml) and p-tosylic acid(9.3 g). The mixture was stirred at 37°C for 3 hours, to which was added under ice-cooling 40 ml of concentrated aqueous ammonia. The mixture was concentrated under reduced pressure, then resulting insolubles were filtered off, followed by purification by means of a $C_8$ silica gel column chromatography (ø5.0 × 6.5 cm, solvent; 5% acetone-water). The product was recrystallized from ethanol to afford 8.4 g of a crystalline compound. The mother liquor of the recrystallization was purified by means of a silica gel chromatography (60 g, solvent; $CHCl_3$/MeOH = 25/1 to 5/1) to afford some amount of a syrupy compound.

The above-mentioned crystals(8.4 g, 27 mmol.) were suspended in a small volume of pyridine. The suspension was subjected to azeotropic distillation under reduced pressure to perform dehydration. The residue was dissolved in 150 ml of anhydrous pyridine. To the solution was added monomethoxytrityl chloride(9 g), and the mixture was left standing for 13 hours at room temperautre. To the resultant was added 20 ml of methanol. The solvent was removed under reduced pressure, and the residue was dissolved in 150 ml of $CHCl_3$. The solution was washed with a 0.1M-TEAB buffer solution(100 ml × 2), then with water(100 ml), and dried(anhydrous sodium sulfate), followed by concentration of $CHCl_3$ under reduced pressure to thereby precipitate crystals of the object compound(9.4 g), m.p.194-195°C. In a similar manner, the above-mentioned syrupy substance was also monomethoxytritylated and combined with the above-mentioned mother liquor. The mixture was dissolved in 70 ml of $CHCl_3$, and the solution was added dropwise to 1 ℓ of n-hexane. Resulting precipitates were collected by filtraiton and dried to afford 10.3 g of the object compound of this Reference Example as a powdery product.

Reference Example 15

Synthesis of 9-[(1R,2S,3R,4R)-4-monomethoxytrityloxymethyl-2,3-(dimethylmethylene)dioxy-cyclopentan-1-yl]-(1-methoxymethylhypoxanthine)

The compound obtained in Reference Example 14 (19.1 g, 33 mmol.) was dissolved in 360 ml of dioxane-dimethylformamide(DMF)(3:1,v/v). To the solution was added, under ice-cooling, 1.47 g of sodium hydride(60%,oil), and the temperature was reverted to room temperature immediately. The mixture was stirred for one hour, to which was added under ice-cooling 3.2 ml of methoxymethyl chloride. The whole mixture was further stirred under ice-cooling for 4 hours. The solvent was removed under reduced pressure, and the residue was dissolved in 200 ml of $CHCl_3$. The solution was washed with 0.1M-TEAB buffer solution(200 ml × 2) then with water(200 ml), followed by drying(anhydrous sodium sulfate). The solvent was then removed under reduced pressure to give a glassy substance. This substance was roughly purified by means of a silica gel chromatography(100 g, solvent;$CHCl_3$/MeOH = 100/1 to 10/1), followed by further purification by means of a $C_8$ silica gel chromatography(ø5.3 × 7.0 cm, solvent; 55 to 90% acetone-water) to afford 16.8 g of a glassy substance, m.p.97 to 102°C.

NMR(90MHz,$CDCl_3$)δppm: 1.28(3H,s,$CH_3$),1.54(3H,s,$CH_3$),2.33-2.60(3H,m,$H_{4'}$,$H_{5'}$),3.10 -3.40(2H,m,$H_{6'}$), 3.43(3H,s,$CH_2OC\underline{H}_3$), 3.79

(3H,s, 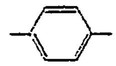$-OCH_3$),

4.47-5.07(3H,m,H,',H₂',H₃'), 5.46(2H,s,N-$CH_2$-O), 6.79(1H,s), 6.88(1H,s), 7.13-7.57(12H,m), 7.82(1H,s), 8.04-(1H,s)

Reference Example 16

Synthesis of 1-[(1R,2S,3R,4R)-4-monomethoxytrityloxymethyl-2,3-(dimethylmethylene)dioxy-cyclopentan-1-yl]-(4-carbamoyl-5-aminoimidazole)

The compound obtained in Reference Example 15 (16.8 g, 26.5 mmol.) was dissolved in ethanol(685 ml). To the solution was quickly added, while heating under reflux, 137 ml of 5M sodium hydroxide heated to about 80°C, and the mixture was refluxed for further 20 minutes. Ethanol was removed under reduced pressure, then the reaction product was extracted with 300 ml of $CHCl_3$. The extract solution was washed with water(300 ml × 2), a 0.1M-TEAB buffer solution (300 ml) and a saturated aqueous saline solution(300 ml), followed by drying(anhydrous sodium sulfate). The resultant was roughly purified by means of a silica gel chromatography (100 g, solvent;$CHCl_3$/MeOH = 100/1 to 50/1), which was subjected to similar silica gel chromatography four times to afford 7.9 g of a colorless glassy substance, m.p.107 to 112°C.

NMR(90MHz,$CDCl_3$)δppm: 1.27(3H,s), 1.58(3H,s), 1.90-2.67(3H,m), 3.23-3.40(2H,m), 3.80(3H,s), 4.07-4.50(3H,m), 5.54(2H,bs,$NH_2$), 6.80(1H,s), 6.21(2H,s), 7.20-7.57(13H,m)

Reference Example 17

Synthesis of 1-[(1R,2S,3R,4R)-4-hydroxymethyl-2,3-(dimethylmethylene)dioxy-cyclopentan-1-yl]-(4-carbamoyl-5-aminoimidazole)(the compound of Reference Example 17-1) and synthesis of 1-[-(1R,2S,3R,4R)-4-hydroxymethyl-2,3-dihydroxyl-cyclopentan-1-yl]-(4-carbamoyl-5-aminoimidazole)(the compound of Reference Example 17-2)

The compound obtained in Reference Example 16 (7.9 g, 13.9 mmol.) was dissolved in 120 ml of 80% acetic acid, and the solution was stirred at 40°C for 7 hours. The solution was concentrated under reduced pressure. To the concentrate was added water, and the mixture was subjected to azeotropic distillation several times. The residue was distiributed to water and ether. The aqueous phase was washed further with ether and concentrated under reduced pressure followed by purification by means of a $C_8$ silica gel chromatography. Fractions eluted with water and 5% aqueous acetone were combined, and the solvent was removed under reduced pressure. The residue was subjected to azeotropic distillation with ethanol twice to allow crystals of the compound of this Reference Example 17-2 to precipitate (0.34 g,m.p.212-214°C).

On the other hand, fractions eluted with 15% aqueous acetone were combined, and the solvent was removed under reduced pressure. The residue was subjected to azeotropic distillaiton with ethanol to afford 1.85 g of crystals of the compound of this Reference Example 17-1 metnioned above, m.p.169-171°C-(decomp.).

Properties of the compound of Reference Example 17-1 Elemental Analysis (%) $C_{13}H_{20}N_4O_4$•$0.20H_2O$ molecular weight 299.928

Calcd. : C; 52.06, H; 6.86, N; 18.68

Found : C; 52.18, H; 6.60, N; 18.63

NMR(100MHz,DMSO-$d_6$)δppm: 1.26(3H,s), 1.50(3H,s), 1.70-2.40(3H,m), 3.40-3.60(2H,m), 4.16-4.82-(4H,m,H ',H₂',H₃',OH), 5.72(2H,bs,$NH_2$), 6.65(2H,bs,$NH_2$), 7.26(1H,s,$H_2$)

Properties of the compound of Reference Example 17-2 Elemental Analysis (%) $C_{10}H_{16}N_4O_4 \bullet 0.2C_2H_5OH \bullet 0.5H_2O$ molecular weight 274.482

Calcd. : C; 45.50, H; 6.63, N; 20.42

Found : C; 45.98, H; 6.32, N; 20.07

NMR(100MHz,DMSO-$d_6$)δppm: 1.00-2.40(3H,m,$H_4'$,$H_5'$), 3.38-3.54(2H,m,$H_6'$), 3.68-4.34(3H,m,$H_1'$,$H_2'$,$H_3'$), 4.56-5.00(3H,m,OH), 5.69(2H,bs,$NH_2$), 6.63(2H,bs,$NH_2$), 7.22(1H,s,$H_2$)

Reference Example 18

Synthesis of 1-[(1R,2S,3R,4R)-4-hydroxymethyl-2,3-(dimethylmethylene)dioxy-cyclopentan-1-yl]-[4-carbamoyl-5-(N-benzoyl-S-methylisothio-carbamoyl)aminoimidazole]

The compound(300 mg, 1 mmol.) of Reference Example 17-1 was suspended in 18 ml of acetone. To the suspension was added dropwise, while heating under reflux, 5 ml of acetone solution containing 150μl of benzoylisothiocyanate over 10 minutes. The reflux was conducted for further one hour. The solvent was removed under reduced pressure, and the residue was dissolved in 5 ml of $CHCl_3$-acetone(1:1,v/v). The solution was added dropwise under stirring to 100 ml of n-hexane. Resulting precipitates were collected by centrifugal separation, then dried to afford a pale yellow powdery compound. This compound was dissolved in 10 ml of 0.2N sodium hydroxide. To the solution was added 110μl of dimethyl sulfate, and the mixture was stirred for 1.5 hour. To the mixture were added, under ice-cooling, several drops of acetic acid (pH 6 to 7). Resulting insolubles were extracted with $CHCl_3$(10 ml × 2), and the extract was washed with water(20 ml × 3), dried(anhydrous sodium sulfate) and purified by means of a silica gel chromatography(11 g, solvent;$CHCl_3$/MeOH = 100/1 to 40/1) to give a colorless glassy substance, which was dissolved in dichloromethane. The solution was added under stirring to 100 ml of n-hexane, then resulting precipitates were collected by centrifugal separation and dried to afford 320 mg of a white powdery compound.

Elemental Analysis (%) $C_{22}H_{27}N_5O_5S_1 \bullet 0.5H_2O$ molecular weight 482.561

Calcd. : C; 54.76, H; 5.85, N; 14.51

Found : C; 54.75, H; 5.40, N; 14.35

λmax(nm): (EtOH);239, 321(sh), ($H^+$);247, 305(sh), ($OH^-$); 264(sh)

NMR(100MHz,$CDCl_3$)δppm: 1.30(3H,s,$CH_3$), 1.54(3H,s,$CH_3$), 2.0-2.52(3H,m,$H_4'$,$H_5'$),2.58(3H,s,S-$CH_3$), 3.75-(2H,d,$H_6'$), 4.48-4.96(3H,m,$H_1'$,$H_2'$,$H_3'$), 5.85(1H,bs,NH),7.58(1H,s,NH), 7.26-7.54(4H,m), 7.80-7.94(2H,m)

Reference Example 19

Synthesis of 9-[(1R,2S,3R,4R)-4-hydroxymethyl-2,3-(dimethylmethylene)dioxy-cyclopentan-1-yl]guanine

The compound(820 mg, 1.75 mmol) obtained in Reference Example 18 was added to 14 ml of 6N sodium hydroxide. The mixture was quickly heated to dissolve under stirring. The heating under reflux was continued for one hour. The resultant solution was neutralized with 1N hydrochloric acid under ice-cooling, followed by rough purification by means of a $C_{18}$silica gel chromatography(10 g, solvent;water and 20% acetone-water). The aqueous solution was washed with $CHCl_3$, concentrated under reduced pressure and crystallized from water to afford 410 mg of the product, m.p.287-288°C.

Elemental Analysis (%) $C_{14}H_{19}N_5O_4 \bullet 0.80H_2O$ Molecular weight 335.747

Calcd. : C; 50.08, H; 6.18, N; 20.86

Found : C; 50.28, H; 5.89, N; 20.86

λmax(nm): ($H_2O$); 254, 274(sh), ($H^+$); 256, 281, ($OH^-$); 258(sh), 270

NMR(100MHz,DMSO-$d_6$ + $D_2O$)δppm: 1.26(3H,s,$CH_3$), 1.51(3H,s,$CH_3$), 1.84-2.40(3H,m,$H_4'$,$H_5'$),3.53-(2H,d,$H_6'$), 4.46-5.04(3H,m,$H_1'$,$H_2'$,$H_3'$), 7.86(1H,s,$H_8$); (100MHz,DMSO-$d_6$)δppm: 3.31(1H,bs,OH), 6.63-(2H,s,$NH_2$), 10.85(1H,bs,NH)

Reference Example 20

Synthesis of 9-[(1R,2S,3R,4R)-4-hydroxymethyl-2,3-dihydroxyl-cyclopentan-1-yl]guanine

The compound obtained in Reference Example 19 (150 mg, 0.46 mmol.) was dissolved in 20 ml of 0.05N-HCl, and the solution was heated at 70°C for 20 minutes.

The reaction solution was neutralized with 1N-NaOH under ice-cooling to afford crystals(91 mg) of the object compound, m.p.268-270°C(decomp.). $[\alpha]_D^{25}$ = -31.4° (c=0.67,DMF)

Elemental Analysis (%) $C_{11}H_{15}N_5O_4 \bullet 0.7H_2O$ molecular weight 293.882

Calcd. : C; 44.96, H; 5.62, N; 23.83

Found : C; 45.19, H; 5.95, N; 23.65

Reference Example 21

Synthesis of 9-[(1R,3S,4R)-4-hydroxymethyl-3-hydroxyl-cyclopentan-1-yl]hypoxanthine

In 200 ml of toluene was dissolved 12.37 g of 9-[(1R,3S,4R)-4-methyl-3,6-(tetraisopropyldisiloxanyl)-dioxy-cyclopentan-1-yl]hypoxanthine. To the solution was added 10 g of tetrabutylammonium fluoride, and the mixture was stirred at 75°C for 2 hours.

The reaction solution was concentrated to dryness, and the concentrate was dissolved in water. The solution was allowed to be adsorbed on a column of activated charcoal (30 g). The substance eluted with 1 ℓ of 50% ethanolic water was recrystallized from methanol containing ether to afford 2.6 g of the object compound as crystals, m.p.170°C

Elemental Analysis $C_{11}H_{14}N_4O_3 \bullet H_2O$

Calcd. : C 49.25 H 6.01 N 20.88

Found : C 49.08 H 5.86 N 20.81

Test Example 1

Inhibitory Effect of Antiviral Compounds against Herpes Simplex Virus Type 1, HF Strainn

| Compound | $ID_{50}$ a) (μg/ml) | Cytotoxicity b) (μg/ml) |
|---|---|---|
| Carbocyclic·2'-deoxy-guanosine-6'-monophosphate | 0.2 | 50 |
| Carbocyclic·2'-deoxy-guanosine-6'-triphosphate | 0.4 | 50 |

Vero cells were infected with 100 $TCID_{50}$(50% tissue-culture infected doses) of viruses and cultured with various concentrations of the compounds. Antiviral activity ($ID_{50}$; 50% inhibitory dose) was observed three days after the infection, when the control culture without any drug showed 100% cytopathogenic effects. Cytotoxicity was observed with the non-infected control culture at the same time.

a) the concentration required to reduce the viral cytopathogenic effect by 50%

b) the minimum concentration of compounds to cause a visible disruption of normal cell morphology

**Claims**

1. A compound of the formula

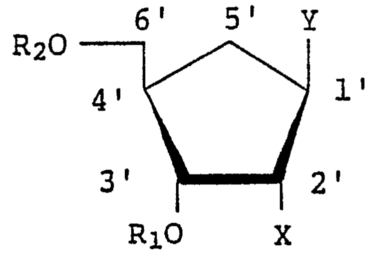

wherein Y stands for a purine base residue or 5-amino-4-carbamoyl-imidazol-1-yl; X stands for hydrogen or an optionally protected hydroxyl group, provided that, when Y is adenin-9-yl, X is hydrogen; $R_1$ and $R_2$ independently stand for hydrogen or a group represented by the formula (A)

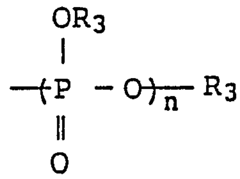
(A)

n being an integer of 1 to 3, provided that $R_1$ and $R_2$ are not both hydrogen, wherein $R_1$ and $R_2$ can combine to form a group of the formula (B)

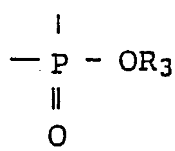
(B)

and wherein $R_3$ stands for hydrogen or a hydrocarbon residue having 1 to 14 carbon atoms, and, when two or more $R_3$'s exist, they may be different from one another, and salts thereof.

2. The compound according to claim 1, wherein Y is a purine base residue which is contained in a natural nucleic acid as a constituent.

3. The compound according to claim 2, wherein the purine base residue is adenin-9-yl, hypoxanthin-9-yl, guanin-9-yl, xanthin-9-yl or 2,6-diaminopurin-9-yl.

4. The compound according to claim 1, wherein $R_1$ is a group represented by the formula (A) and $R_2$ is hydrogen.

5. The compound according to claim 1, wherein $R_1$ is hydrogen and $R_2$ is a group represented by the formula (A).

6. The compound according to claim 1, wherein $R_1$ and $R_2$, combinedly, form a group represented by the formula (B).

7. The compound according to claim 1, wherein Y is guanin-9-yl, X is hydrogen or hydroxyl, $R_1$ is hydrogen and $R_2$ is the group represented by the formula (A) wherein $R_3$ is hydrogen.

8. The compound according to claim 7, wherein n of the group represented by the formula (A) is 1.

9. The compound according to claim 7, wherein n of the group represented by the formula (A) is 3.

10. The compound according to claim 1, wherein Y is guanin-9-yl, X is hydrogen or hydroxyl and $R_1$ and $R_2$, combinedly, form a group of the formula (B).

11. The compound according to claim 1, wherein Y is adenin-9-yl, X is hydrogen and $R_1$ and $R_2$, combinedly, form a group of the formula (B).

12. The compound according to claim 1, wherein Y is 5-amino-4-carbamoyl-imidazol-1-yl, X is hydroxyl and $R_1$ and $R_2$, combinedly, form a group of the formula (B).

13. A method of preparing a compound of the formula (I)

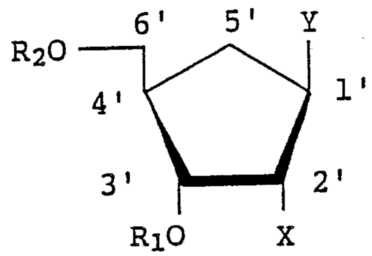

(I)

wherein Y stands for a purine base residue or 5-amino-4-carbamoyl-imidazol-1-yl; X stands for hydrogen or an optionally protected hydroxyl group provided that, when Y is adenin-9-yl, X is hydrogen; $R_1$ and $R_2$ independently stand for hydrogen or a group represented by the formula (A)

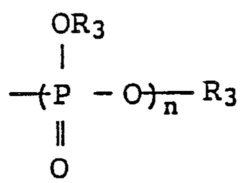

(A)

n being an integer of 1 to 3, provided that $R_1$ and $R_2$ are not both hydrogen, wherein $R_1$ and $R_2$ can combine to form a group of the formula (B)

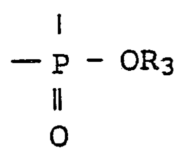

(B)

and wherein $R_3$ stands for hydrogen or a hydrocarbon residue having 1 to 14 carbon atoms, and, when two or more $R_3$'s exist, they may be different from one another, or salts thereof, which comprises subjecting a compound represented by the formula (II)

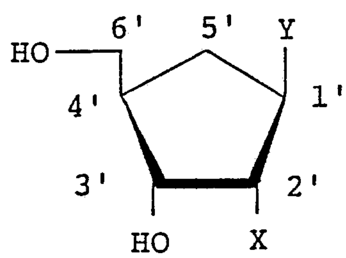

(II)

wherein Y and X are of the same meaning as defined above to phosphorylation and hydrolysis.

14. The method according to claim 13, wherein a compound of the formula (I) wherein $R_1$ has a group represented by the formula (A) and $R_2$ is hydrogen is prepared by protecting the hydroxyl group at 6'-position of the starting compound, then subjecting to the phosphorylation with a phosphorylating agent of about 2.5 to 10 times the theoretical amount in number of moles at 10 to 30°C and hydrolysis in ice water.

15. The method according to claim 13, wherein a compound of the formula (I) wherein $R_2$ has a group represented by the formula (A) and $R_1$ is hydrogen is prepared by protecting or without protecting the hydroxyl group at 3'-position of the starting compound, then subjecting to the phosphorylation with a phosphorylating agent of about 1.2 to 1.5 times the theoretical amount in number of moles at -40°C to 10°C and hydrolysis in ice water.

16. The method according to claim 13, wherein a compound of the formula (I) wherein $R_1$ and $R_2$ combinedly form a group of the formula (B) is prepared by subjecting a compound of the formula (I) wherein $R_1$ has a group represented by the formula (A) and $R_2$ is hydrogen, or $R_2$ has a group represented by the formula (A) and $R_1$ is hydrogen to cyclization.

17. An antivirus agent containing an effective virucidal amount of a compound represented by the formula

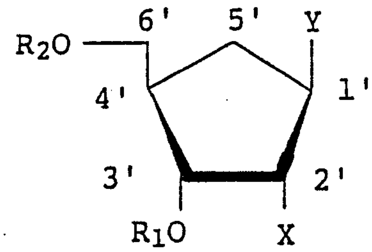

wherein Y stands for a purine base residue or 5-amino-4-carbamoyl-imidazol-1-yl; X' stands for hydrogen or hydroxyl group, provided that, when Y is adenin-9-yl, X is hydrogen; $R_1$ and $R_2$ independently stand for hydrogen or a group represented by the formula

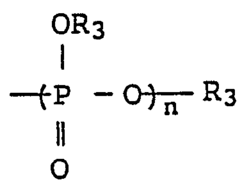

n being an integer of 1 to 3, provided that $R_1$ and $R_2$ are not both hydrogen, wherein $R_1$ and $R_2$ can combine to form a group of the formula (B)

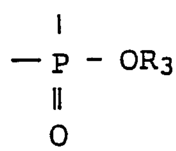

and wherein $R_3$ stands for hydrogen or a hydrocarbon residue having 1 to 14 carbon atoms, and, when two or more $R_3$'s exist, they may be different from one another, and salts thereof and a pharmacologically acceptable carrier.

18. A method of preparing an antiviral composition which comprises incorporating therein an effective amount of a compound represented by the formula

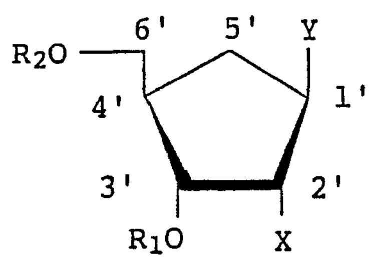

wherein Y stands for a purine base residue or 5-amino-4-carbamoyl-imidazol-1-yl; X' stands for hydrogen or hydroxyl group, provided that, when Y is adenin-9-yl, X is hydrogen; $R_1$ and $R_2$ independently stand for hydrogen or a group represented by the formula

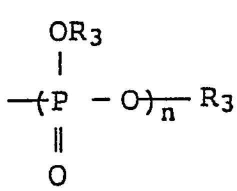

n being an integer of 1 to 3, provided that $R_1$ and $R_2$ are not both hydrogen, wherein $R_1$ and $R_2$ can combine to form a group of the formula (B)

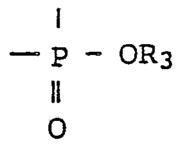

and wherein $R_3$ stands for hydrogen or a hydrocarbon residue having 1 to 14 carbon atoms, and, when two or more $R_3$'s exist, they may be different from one another, and salts thereof.